# DEMANDE DE BREVET EUROPEEN

(11) **EP 4 332 571 A1**
(43) Date de publication de la demande: **06.03.2024**
(21) Numéro de dépôt: 23193359.9
(22) Date de dépôt: 25.08.2023
(51) Int. Cl.: G01N 33/483, G01N 21/17, G02F 1/1343

(54) **STRUCTURE D'INTERCONNEXION CONDUCTRICE ET TRANSPARENTE, PROCÉDÉ DE FABRICATION ET SYSTÈME ASSOCIÉS**

(30) Priorité: 29.08.2022 GM 861922
(71) Demandeur: Commissariat à l'énergie atomique et aux énergies alternatives, 75015 Paris (FR)
(72) Inventeur: RACINE, Benoit, 38054 GRENOBLE CEDEX 09 (FR); HAON, Olivier, 38054 GRENOBLE CEDEX 09 (FR)
(74) Mandataire: Cabinet Camus Lebkiri

(57) **Abrégé**

Un aspect de l'invention concerne une structure d'interconnexion (1) comprenant :
- un substrat (10) formé d'un premier matériau électriquement isolant et optiquement transparent, le substrat (10) comprenant une première face (11) et une deuxième face (12) opposée, la première face (11) définissant un plan du substrat ;
- une pluralité d'électrodes transparentes (20) ;
ladite structure d'interconnexion (1) étant remarquable en ce que les électrodes transparentes (20) traversent le substrat (10) de la première face (11) jusqu'à la deuxième face (12) du substrat (10) parallèlement les unes aux autres, et sont isolées électriquement les unes des autres par le premier matériau électriquement isolant et optiquement transparent.

## Description

### DOMAINE TECHNIQUE DE L'INVENTION

Le domaine technique de l'invention est celui de l'interconnexion électrique, optique et mécanique entre un composant électronique et un échantillon à analyser, par exemple un échantillon biologique tel que des cellules neuronales en culture dans une cellule microfluidique.

L'invention trouve une application dans les domaines de la biologie et de la santé, en particulier dans le domaine des organes sur puces, ou « organ-on-chip » en anglais.

### ARRIÈRE-PLAN TECHNOLOGIQUE DE L'INVENTION

Les organes sur puce sont des plateformes technologiques miniaturisées dans lesquelles des cellules sont cultivées *in vitro* et combinées à des technologies microfluidiques et microélectroniques ainsi qu'à des capteurs, ces capteurs pouvant être des capteurs électroniques et/ou optiques. Les organes sur puces permettent de reproduire et d'étudier le fonctionnement des organes au plus près de leur fonctionnement dans le corps humain.

Appliqués aux cellules neuronales (ou « neurones »), les organes sur puce offrent la possibilité de comprendre certaines maladies neuro-dégénératives, telles que la maladie de Parkinson ou la maladie d'Alzheimer, et de tester in-vitro des traitements introduits via le circuit microfluidique.

La mesure en temps réel de l'activité électrique des cellules neuronales est l'une des voies d'accès à la compréhension de ces maladies. L'activité électrique des cellules neuronales se traduit par l'émission de potentiels d'action de l'ordre de la dizaine de millivolts, par exemple 70 mV. Ces potentiels d'action peuvent être reçus soit directement par un capteur électronique sur lequel sont déposées les cellules neuronales, soit via un dispositif d'interfaçage disposé entre les cellules neuronales et un capteur électronique.

Pour mesurer directement les potentiels d'action d'une cellule neuronale, il est connu de mettre en contact une cellule neuronale avec une surface recouverte d'un diélectrique fin et connectée à la grille d'un transistor MOS (acronyme de « Metal Oxyde Semiconductor » en anglais). Lorsque la cellule neuronale est stimulée électriquement, elle émet des potentiels d'action qui sont couplés de façon capacitive au transistor. En d'autres termes, le transistor agit comme un capteur de tension capacitif capable d'effectuer des enregistrements de l'activité électrique d'une cellule individuelle.

Un capteur électronique appelé « Multi Transistor Array » en anglais a été conçu sur cette base. Celui-ci comporte une pluralité de transistors MOS connectés en série et formant ainsi un réseau ou matrice de transistors.

La taille des transistors et le pas du réseau de transistors garantissent que chaque cellule neuronale est électriquement mesurée individuellement, quelle que soit sa position sur la surface de la matrice. Le réseau effectue des enregistrements de l'activité électrique des cellules en parallèle. Un tel capteur fonctionne donc comme un dispositif d'imagerie électrophysiologique dont la résolution spatiale est à l'échelle de la cellule neuronale.

Une telle matrice de transistors MOS comportant 16384 transistors agencés en série pour former une surface d'analyse de 1 mm² a, par exemple, été élaborée. Un tel dispositif permet de faire croître ou de cultiver des cellules neuronales sur les transistors et de mesurer l'activité électrique de ces cellules.

L'inconvénient de ce type de capteur est qu'il comprend des transistors formés sur des substrats en silicium, donc opaques à la lumière visible. C'est une contrainte importante lorsque l'on souhaite observer ou mesurer l'activité des cellules neuronales par voie optique, c'est-à-dire par microscopie ou tout autre mode d'imagerie utilisant de la lumière visible, pour bénéficier de la complémentarité entre les méthodes de caractérisation optiques et électriques. Plus précisément, à cause de l'opacité des transistors, la seule modalité d'imagerie optique possible est la modalité de réflexion (ou « épi ») qui consiste à mesurer le signal réfléchi par l'échantillon. Cette modalité limite le type de signaux optiques pouvant être mesurés sur les cellules.

Pour y remédier, il est possible d'utiliser un dispositif d'interconnexion entièrement transparent à la lumière visible. De façon générale, un tel dispositif ne comprend pas de transistors mais des zones conductrices - dites électrodes - reliant électriquement et passivement des points de contacts d'entrée et des points de contacts de sortie. Ainsi, lorsque des cellules neuronales sont disposées sur les points de contact d'entrée, leur activité électrique peut être recueillie et transmise aux contacts de sortie, puis traitée par les capteurs externes connectés aux contacts de sortie.

Un tel dispositif est décrit dans le brevet EP2245454B1 pour la mesure de l'activité électrique d'un échantillon biologique. Il s'agit d'un réseau de microélectrodes passives présentant des caractéristiques de transparence et de biocompatibilité.

Le réseau de microélectrodes est dit passif dans le sens où il comprend des agencements de circuits de microélectrodes utilisés uniquement pour la transduction des signaux bioélectriques, c'est-à-dire sans autre prétraitement. Les matériaux utilisés pour le substrat et le réseau de microélectrodes sont des polymères transparents, ce qui permet l'observation par voie optique de l'échantillon à travers ledit réseau.

Cependant, un tel réseau ne permet pas de transmettre une image de l'activité électrique produite par l'échantillon de façon directe et simple. En effet, il est difficile de faire une correspondance entre un signal de sortie délivré par un contact de sortie et la partie de l'échantillon électriquement actif. Il faut pour y arriver prévoir de connecter les contacts de sortie par une liaison par fil (ou similaire) et prendre en compte le plan de routage donnant la correspondance entre chaque contact de sortie et la position de l'électrode de mesure dans le réseau.

Il existe donc un besoin pour un dispositif d'interconnexion transparent permettant d'imager de façon simple et directe l'activité électrique de l'échantillon.

Ce besoin existe pour l'étude d'un échantillon de cellules neuronales, et plus largement pour l'étude d'échantillons comprenant au moins une source apte à générer un signal électrique.

### RÉSUMÉ DE L'INVENTION

L'invention offre une solution au problème évoqué précédemment en proposant une structure d'interconnexion transparente avec une structure d'électrodes traversantes permettant de relier électriquement un contact de sortie à un unique contact d'entrée en conservant les informations spatiales du contact d'entrée, en particulier sa position par rapport aux autres contacts.

Un premier aspect de l'invention concerne une structure d'interconnexion comprenant :
- un substrat formé d'un premier matériau électriquement isolant et optiquement transparent, le substrat comprenant une première face et une deuxième face opposée, la première face définissant un plan du substrat ;
- une pluralité d'électrodes transparentes ;
ladite structure d'interconnexion étant remarquable en ce que les électrodes transparentes traversent le substrat de la première face jusqu'à la deuxième face du substrat parallèlement les unes aux autres, et sont isolées électriquement les unes des autres par le premier matériau électriquement isolant et optiquement transparent.

Ainsi, une pluralité d'électrodes transparentes, dites électrodes traversantes, traversent le substrat entre les première et deuxième faces, c'est-à-dire dans le sens de son épaisseur, tout en étant isolées électriquement les unes des autres par le substrat.

Les extrémités des électrodes traversantes forment, sur ou dans la première face et sur ou dans la deuxième face du substrat, des contacts électriques pouvant recevoir ou restituer une tension. Chaque contact électrique de la première face est relié à un unique contact électrique de la deuxième face, tout en conservant les informations spatiales relatives au contact électrique de la première face. Par informations spatiales, on entend la position du contact par rapport aux autres contacts de la face. Autrement dit, deux contacts électriques reliés occupent chacun sur leur face sensiblement la même position par rapport aux autres contacts. Il y a donc une correspondance spatiale entre les contacts électriques de la première face et les contacts électriques de la deuxième face du substrat, ce qui permet de transmettre fidèlement et directement, c'est-à-dire sans avoir recours à un traitement, à un dispositif électronique externe ou à une connexion filaire, une image électrique formée sur la première face vers la deuxième face.

Par ailleurs, lorsque la première face du substrat est éclairée par une onde lumineuse incidente dans le domaine du visible, il est possible de détecter une onde lumineuse transmise directement en regard des contacts électriques de la deuxième face, cela sans que la structure des électrodes ou du substrat ne perturbe sensiblement la détection. En effet, les électrodes et le substrat sont optiquement transparents, c'est-à-dire qu'ils présentent un coefficient de transmission optique supérieur à 85% pour au moins une longueur d'onde de la bande spectrale 400-800nm.

La structure d'interconnexion permet donc de transmettre de façon simultanée et superposée une image électrique et une onde lumineuse transmise à travers le substrat. Autrement dit, la structure d'interconnexion selon l'invention permet de combiner des imageries optique et électrique et, ainsi, de bénéficier de leur complémentarité.

Avantageusement, chaque électrode transparente comprend une première portion disposée sur la première surface du substrat.

La première portion permet d'obtenir une extrémité d'électrode compacte, exempte de toute cavité, vide ou creux, ces irrégularités pouvant dégrader à la fois la conductivité de l'électrode et la qualité du couplage électrique de ladite électrode avec une source de tension, typiquement un échantillon biologique.

Ainsi, la première portion permet un meilleur contact électrique entre l'électrode et la source de tension.

Enfin, la première portion permet d'étendre latéralement les dimensions de l'électrode, de sorte à augmenter la surface de contact électrique et/ou à adapter la résolution spatiale électrique.

Avantageusement, la première portion présente des dimensions comprises entre 6 µm et 55 µm dans le plan du substrat, et une épaisseur comprise entre 5 nm et 100 nm.

Ainsi, la résolution spatiale est micrométrique, par exemple de l'ordre de 10 µm.

Avantageusement, chaque électrode transparente comprend en outre une deuxième portion disposée sur la deuxième face du substrat en regard de la première portion.

La deuxième portion permet d'améliorer la conductivité de l'électrode en obtenant une deuxième extrémité d'électrode compacte, exempte de toute cavité, vide ou creux. La deuxième portion permet aussi d'améliorer le contact électrique entre l'électrode et un récepteur de tension, typiquement un capteur électronique ou un dispositif électro-optique.

Avantageusement, la deuxième portion présente des dimensions comprises entre 6 µm et 55 µm dans un plan parallèle au plan du substrat, et une épaisseur comprise entre 5 nm et 100 nm.

Avantageusement, les électrodes transparentes sont symétriques par rapport à un plan parallèle au plan du substrat.

Cela permet d'améliorer la fidélité de l'image électrique transmise par la structure d'interconnexion. Lorsque la structure d'interconnexion comprend une première et une deuxième portion, celles-ci ont alors des sections identiques (dans un plan parallèle au substrat). Par sections identiques, on entend que la section de la première portion et la section de la deuxième portion sont de même forme (circulaire, carrée, rectangulaire, hexagonale, etc.) et de mêmes dimensions. Ainsi, il y a une correspondance spatiale, à la fois en position et en dimensions, entre les contacts électriques de la première face et les contacts électriques de la deuxième face.

De préférence, chaque électrode transparente comprend une portion cylindrique creuse s'étendant de la première face jusqu'à la deuxième face du substrat, la structure d'interconnexion comprenant en outre un noyau transparent disposé à l'intérieur de la portion cylindrique creuse de chaque électrode transparente.

De préférence, la portion cylindrique creuse présente dans un plan parallèle au plan du substrat des dimensions extérieures comprises entre 5 µm et 50 µm, et comprend une paroi d'épaisseur comprise entre 5 nm et 100 nm.

Dans un premier mode de réalisation, le noyau transparent est formé d'un matériau électriquement conducteur et optiquement transparent.

Cette caractéristique permet d'optimiser la conductivité de l'électrode tout en garantissant la transparence de ladite électrode. Dans ce premier mode de réalisation, le noyau transparent fait partie de l'électrode.

Dans un deuxième mode de réalisation, le noyau transparent est formé d'un deuxième matériau électriquement isolant et optiquement transparent.

Cette caractéristique permet d'optimiser la transparence de l'électrode tout en garantissant une conductivité satisfaisante de ladite électrode. Dans ce deuxième mode de réalisation, le noyau transparent ne fait pas partie de l'électrode.

La structure d'interconnexion selon le premier aspect de l'invention peut également présenter une ou plusieurs caractéristiques ci-dessous, considérées individuellement ou selon toutes les combinaisons techniquement possibles.

Le premier matériau électriquement isolant et optiquement transparent peut être un verre, une résine, un polymère organo-minéral ou un polystyrène expansé.

Le substrat peut présenter entre la première face et la deuxième face du substrat une épaisseur comprise entre 50 µm et 300 µm.

La structure d'interconnexion peut ainsi présenter une rigidité mécanique suffisante pour recevoir un échantillon biologique encapsulé dans une chambre fluidique ou micro-fluidique.

Le substrat peut présenter des dimensions comprises entre 2,5 cm et 10 cm dans un plan parallèle au plan du substrat.

Un tel substrat permet d'obtenir une ou plusieurs surfaces actives de grande taille, c'est-à-dire de l'ordre du cm². Par surface active, on désigne la surface couverte par plusieurs électrodes transparentes et traversantes relativement à la surface du substrat.

Les électrodes transparentes peuvent être espacées les unes des autres à l'intérieur du substrat par une distance bord-à-bord comprise entre 5 µm et 30 µm.

Ainsi la résolution spatiale est micrométrique.

Avantageusement, les électrodes transparentes sont identiques en taille et en forme et présentent dans le plan du substrat un premier pas de répétition dans une première direction et un deuxième pas de répétition dans une deuxième direction sécante à la première direction.

Le premier pas de répétition et le deuxième pas de répétition peuvent être identiques ou non.

Les électrodes transparentes et traversantes forment ainsi un réseau matriciel, ce qui permet de faciliter la fabrication et l'utilisation de la structure d'interconnexion. Par exemple, la structure d'interconnexion est rendue directement compatible avec des capteurs matriciels.

Les électrodes transparentes peuvent être formées d'un polymère conducteur transparent ou d'un oxyde métallique conducteur transparent tel que le dioxyde d'étain.

Le dioxyde d'étain (SnO2) trouve un avantage particulier lors de la fabrication car il est compatible avec une méthode de dépôt conforme, ce qui n'est pas le cas d'un matériau conducteur et transparent comme l'oxyde d'indium-étain (ITO).

Un deuxième aspect de l'invention concerne un procédé de fabrication d'une structure d'interconnexion, comprenant les étapes suivantes :
- Fournir un substrat formé d'un premier matériau électriquement isolant et optiquement transparent, le substrat comprenant une première face et une deuxième face opposée, la première face définissant un plan du substrat ;
- Aménager dans le substrat une pluralité de cavités, dites cavités traversantes, traversant le substrat entre la première face et la deuxième face du substrat parallèlement les unes aux autres, et étant espacées les unes des autres,
- Former dans les cavités traversantes une pluralité d'électrodes transparentes, lesdites électrodes transparentes traversant le substrat de la première face jusqu'à la deuxième face du substrat parallèlement les unes aux autres, et étant isolées électriquement les unes des autres par le premier matériau électriquement isolant et optiquement transparent.

De préférence, l'étape de formation de la pluralité d'électrodes transparentes comprend les étapes suivantes :
- Déposer une première couche de matériau électriquement conducteur et optiquement transparent sur :
   - la surface interne de chacune des cavités traversantes,
   - la première face du substrat, et
   - la deuxième face du substrat ;
- Remplir les cavités traversantes avec un matériau transparent et former une surépaisseur du matériau transparent sur la première et la deuxième face du substrat ;
- Retirer la surépaisseur du matériau transparent ainsi que des portions de la première couche de matériau électriquement conducteur et optiquement transparent déposées sur la première face et la deuxième face du substrat.

De préférence, l'étape de dépôt de la première couche de matériau électriquement conducteur et optiquement transparent est réalisée avec une méthode de dépôt conforme telle que la méthode de dépôt par couches atomiques (ALD, acronyme de « Atomic Layer Déposition » en anglais).

De préférence, l'étape de formation des électrodes transparentes comprend une étape supplémentaire de formation, pour chacune des électrodes traversantes formées, d'une première portion d'électrode transparente sur la première face du substrat et d'une deuxième portion d'électrode transparente sur la deuxième face du substrat, ladite étape supplémentaire comprenant les sous-étapes suivantes :
- déposer une deuxième couche de matériau électriquement conducteur et optiquement transparent sur la première face et la deuxième face du substrat,
- graver la deuxième couche de matériau électriquement conducteur et optiquement transparent dans des régions de la première et deuxième face du substrat, les régions étant situées autour des électrodes transparentes formées.

Avantageusement, l'étape de dépôt de la deuxième couche de matériau conducteur et optiquement transparent est réalisée avec une méthode de dépôt compatible avec un oxyde conducteur transparent (TCO, acronyme de « Transparent Conductive Oxyde » en anglais).

Un troisième aspect de l'invention est un système d'analyse d'un échantillon comprenant :
- une source de lumière produisant une onde lumineuse, dite onde lumineuse incidente,
- une structure d'interconnexion selon le premier aspect de l'invention, la première face du substrat étant disposée en regard de la source de lumière et étant destinée à recevoir un échantillon comprenant au moins une source apte à produire un signal électrique, dit signal électrique de l'échantillon, le signal électrique de l'échantillon étant transmis de la première face du substrat jusqu'à la deuxième face du substrat par l'électrode traversante située en regard de ladite source,
- un dispositif électro-optique disposé en regard de la deuxième face du substrat,
le dispositif électro-optique présentant une pluralité d'électrodes de commande disposées en regard de la pluralité d'électrodes traversantes de la structure d'interconnexion, chaque électrode de commande de la pluralité d'électrodes de commande étant connectée à une électrode traversante de la pluralité d'électrodes traversantes, de sorte que le dispositif électro-optique module l'onde lumineuse transmise par l'échantillon sous l'effet du signal électrique de l'échantillon.

Selon un mode de réalisation préférentiel du troisième aspect de l'invention, le dispositif électro-optique est une cellule de cristaux liquides.

Il est ainsi possible de caractériser l'activité électrique de l'échantillon de manière indirecte en utilisant la complémentarité des modes d'imagerie optique et électrique. Cet aspect de l'invention trouve un avantage particulier pour l'étude de l'activité électrique de cellules neuronales en culture, et plus généralement pour la réalisation d'organes sur puces.

L'invention et ses différentes applications seront mieux comprises à la lecture de la description qui suit et à l'examen des figures qui l'accompagnent.

### BRÈVE DESCRIPTION DES FIGURES

Les figures sont présentées à titre indicatif et nullement limitatif de l'invention.
- La figure 1 représente schématiquement, en vue de dessus, un premier mode de réalisation de la structure d'interconnexion ;
- La figure 2 est une vue en coupe de la structure d'interconnexion selon le premier mode de réalisation ;
- La figure 3 est une vue en coupe de la structure d'interconnexion selon un deuxième mode de réalisation ;
- La figure 4 est un schéma synoptique illustrant l'enchaînement des étapes du procédé de fabrication de la structure d'interconnexion ;
- La figure 5 est un schéma synoptique illustrant un mode de mise en oeuvre préférentiel de la troisième étape du procédé de fabrication ;
- La figure 6 est un schéma synoptique illustrant un mode de mise en oeuvre préférentiel de la quatrième sous-étape de la troisième étape du procédé de fabrication ;
- Les figures 7A à 7G représentent en vue de coupe des étapes ou sous-étapes de fabrication de la structure d'interconnexion ;
- La figure 8 représente schématiquement un système d'analyse d'un échantillon, comprenant la structure d'interconnexion ;
- La figure 9 est une vue agrandie de la figure 8 centrée sur la structure d'interconnexion dans le système d'analyse et sur un échantillon disposé sur la structure d'interconnexion.

### DESCRIPTION DÉTAILLÉE

Les figures sont présentées à titre indicatif et nullement limitatif de l'invention.

Sauf précision contraire, un même élément apparaissant sur des figures différentes présente une référence unique.

Dans la description, le terme « optiquement transparent », ou « transparent », est dit de tout matériau ou élément qui présente un coefficient de transmission optique supérieur à 85% pour au moins une longueur d'onde comprise dans la bande spectrale s'étendant dans le domaine du visible, soit entre 400 nm et 800 nm.

Un premier aspect de l'invention concerne une structure d'interconnexion 1 optiquement transparente comprenant une pluralité d'électrodes traversantes 20.

La structure d'interconnexion 1 est par exemple destinée à s'interfacer entre un échantillon biologique et un dispositif électronique ou électro-optique pour imager l'activité électrique et/ou optique dudit échantillon.

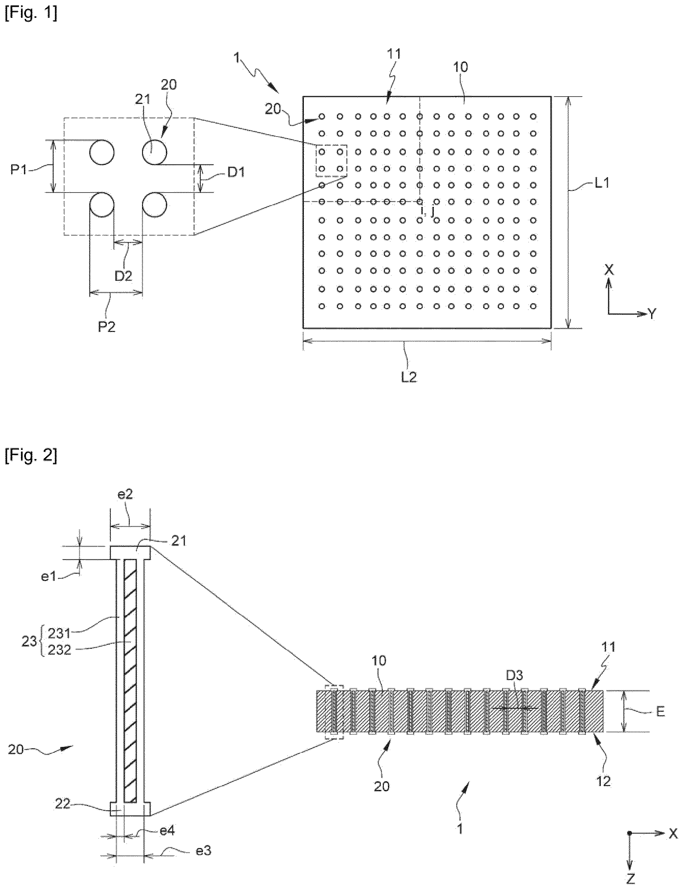

La structure d'interconnexion 1 est décrite à l'appui des figures 1 à 3 qui représentent schématiquement, en vue de dessus et en vue de coupe, deux modes de réalisation de la structure d'interconnexion 1. La vue de dessus (Fig.1) est commune aux deux modes de réalisation.

En référence aux figures 1, 2 et 3, la structure d'interconnexion 1 comprend un substrat isolant et transparent 10 présentant une première face 11 et une deuxième face 12 opposée à la première face 11 et une pluralité d'électrodes transparentes et traversantes 20. On désignera par plan du substrat le plan défini par la première face 11 du substrat 10.

Les première et deuxième faces 11, 12 du substrat 10 sont, dans ces modes de réalisation particuliers, interchangeables car la structure d'interconnexion 1 est symétrique selon un plan parallèle plan du substrat.

Dans la description qui suit, on distinguera ces deux faces uniquement par le fait que la première face 11 du substrat 10 est destinée à recevoir l'échantillon, la deuxième face 12 étant alors destinée à être connectée à un dispositif électronique ou à un dispositif électro-optique.

Les termes « latéral » ou « latéralement » se réfèrent aux axes X et Y tels que représentés figure 1, ces axes X et Y définissant le plan du substrat. Par ailleurs on désignera par « surface couverte par les électrodes » ou « surface active » la surface couverte par les électrodes dans le plan du substrat.

Enfin, les électrodes transparentes et traversantes 20 seront désignées plus simplement par le nom d'électrodes traversantes 20 ou encore électrodes 20.

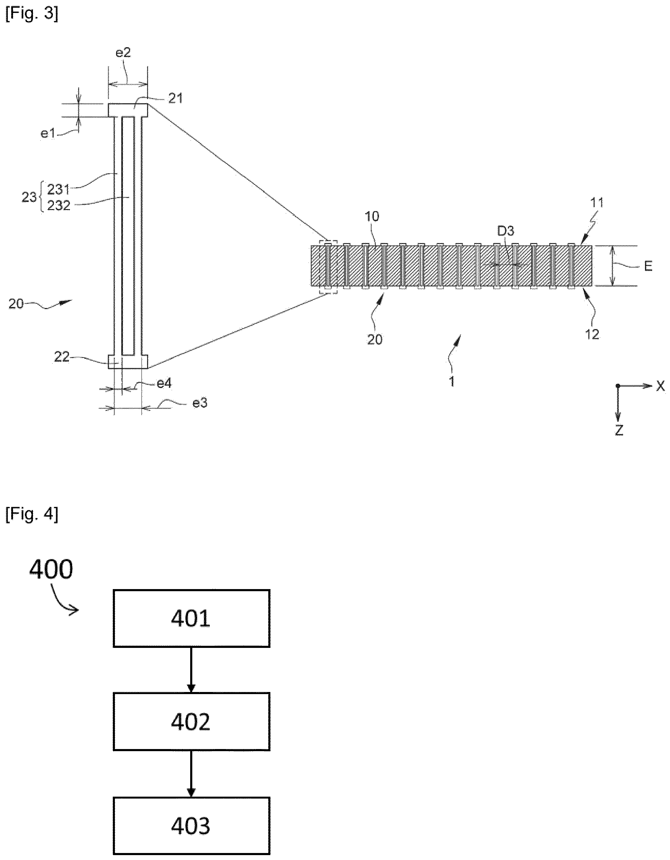

Le substrat 10 présente une épaisseur E entre la première face 11 et la deuxième face 12, et des dimensions latérales L1 et L2. Les dimensions latérales L1 et L2 peuvent être comprises entre 2,5 cm et 10 cm, afin d'obtenir une ou plusieurs surfaces actives (correspondant à un ou plusieurs groupes d'électrodes) de l'ordre du cm². L'épaisseur E du substrat 10 (et l'épaisseur de tout autre élément de la structure d'interconnexion, sauf mention contraire) est mesurée perpendiculairement au plan du substrat, selon un axe Z représenté figure 2 et 3.

Le substrat 10 est formé d'un premier matériau isolant et optiquement transparent.

Le substrat 10 est ainsi avantageusement formé d'un verre, d'une résine, d'un polymère organo-minéral tel que du polydiméthylsiloxane (ou PDMS), d'un polystyrène expansé ou de tout autre matériau à la fois isolant, optiquement transparent et offrant une rigidité mécanique suffisante pour recevoir sur la première face 11 une chambre fluidique permettant de maintenir l'échantillon dans un milieu liquide.

L'épaisseur E du substrat 10 est choisie de manière à conférer une bonne rigidité mécanique au substrat 10. Une bonne rigidité mécanique signifie que le substrat 10 ne se déforme pas lorsqu'il est en interaction avec l'échantillon. Selon le matériau utilisé, l'épaisseur E du substrat 10 peut être comprise entre 50 µm et 300 µm.

Par exemple, le substrat 10 est un verre d'épaisseur E de 300 µm et de dimensions latérales L1 = L2 = 2,4 cm offrant une surface active pouvant atteindre 5,76 cm².

Les électrodes traversantes 20 sont des zones conductrices et optiquement transparentes. Par zone conductrice, on entend une zone formée d'un matériau conducteur permettant de conduire sensiblement sans pertes des tensions allant de quelques millivolts à quelques microvolts. Ces ordres de grandeurs sont typiques des tensions, dites potentiels évoqués, produites par des échantillons biologiques telles que des cellules neuronales. Les électrodes traversantes 20 sont formées d'un matériau, polymère ou oxyde, à la fois conducteur et optiquement transparent, par exemple le Poly(3,4-ethylenedioxythiophene) (ou PEDOT), l'oxyde d'indium-étain (ou ITO pour « Indium Tin Oxide » en anglais), le dioxyde d'étain (SnO₂), l'oxyde de zinc (ZnO) ou l'oxyde de zinc dopé aluminium (ou AZO).

Les électrodes traversantes 20 traversent le substrat sur toute son épaisseur E, de la première face 11 jusqu'à la deuxième face 12 du substrat 10 et sont séparées entre elles par le premier matériau isolant et optiquement transparent du substrat 10. Le premier matériau isolant et optiquement transparent du substrat 10 enrobe (latéralement) les électrodes traversantes 20.

Les électrodes traversantes 20 s'étendent parallèlement les unes aux autres, de préférence dans une direction sensiblement perpendiculaire au plan du substrat, et plus préférentiellement encore selon l'axe Z. Par direction sensiblement perpendiculaire, on entend perpendiculaire à une tolérance de quelques degrés, par exemple 5 degrés (90° ± 5°).

A l'intérieur du substrat 10, la distance D3 entre deux électrodes transparentes 20 voisines, mesurée bord-à-bord, est de préférence comprise entre 5 µm et 30 µm. Sur les figures 2 et 3, la distance D3 est mesurée selon la direction X.

Les figures 2 et 3 montrent également en vue de coupe schématique un agrandissement d'une électrode traversante 20 selon respectivement un premier mode de réalisation et un deuxième mode de réalisation de la structure d'interconnexion 1.

De façon commune à ces deux modes de réalisation, l'électrode traversante 20 comprend préférentiellement, en plus d'une portion intérieure 23 s'étendant de la première face 11 jusqu'à la deuxième face 12 du substrat 10, une première portion 21 disposée sur la première face 11 du substrat 10.

L'électrode traversante 20 peut aussi comprendre une deuxième portion 22 disposée sur la deuxième face 12 du substrat 10 en regard de la première portion 21. La première portion 21 et la deuxième portion 22 prolongent dans ce cas la portion intérieure 23 et forment les extrémités de l'électrode 20. La première portion 21 et la deuxième portion 22 sont en contact direct avec la portion intérieure de sorte à assurer la continuité électrique de l'électrode 20 d'une face à l'autre du substrat 10.

La première portion 21 est une couche mince d'un matériau électriquement conducteur et optiquement transparent choisi parmi les matériaux électriquement conducteurs et optiquement transparents cités précédemment. Ce peut être le même matériau ou un matériau différent de celui formant la portion intérieure 23 de l'électrode traversante 20.

Une couche mince désigne ici une couche d'épaisseur comprise entre 5 nm et 100 nm. Ainsi, l'épaisseur e1 de la première portion 21 est comprise entre 5 nm et 100 nm.

Latéralement, la première portion 21 s'étend avantageusement au-delà de la portion intérieure 23 de l'électrode 20 (à l'intérieur du substrat 10), augmentant ainsi la surface de contact de l'électrode 20 par rapport à une portion intérieure 23 seule. La première portion 21 présente de préférence des dimensions latérales strictement supérieures aux dimensions latérales de la portion intérieure 23. Ainsi, la première portion 21 peut présenter des dimensions latérales comprises entre 6 µm et 55 µm, pour des dimensions latérales de la portion intérieure 23 comprises entre 5 µm et 50 µm. Par exemple, sur les figures 2 et 3, la première portion 21 occupe une surface circulaire de diamètre e2 compris entre 6 µm et 55 µm, tandis que la portion intérieure 23 de l'électrode 20 a un diamètre e3 compris entre 5 µm et 50 µm.

La première portion 21 peut avoir, dans le plan du substrat 11, une section de forme circulaire ou une autre forme, par exemple une forme carrée, rectangulaire, hexagonale, etc.

En se conformant à la portion intérieure 23, la première portion 21 permet de combler d'éventuels creux, trous ou vides laissés en surface de la portion intérieure 23 lors de la fabrication des électrodes. En cela, la première portion 21 contribue à réaliser une électrode traversante 20 exempte de creux et offrant donc une meilleure conductivité électrique. La première portion 21 permet en outre d'étendre la surface du contact électrique tout en réduisant les irrégularités de surface de ce contact. Le couplage électrique avec l'échantillon est ainsi amélioré.

La deuxième portion 22 est similaire à la première portion 21 ; la description, les effets et avantages donnés pour la première portion 21 s'appliquent donc à la deuxième portion 22. Le matériau conducteur et optiquement transparent choisi pour former la deuxième portion 22 peut être le même ou différent du matériau choisi pour former la première portion 21.

La première portion 21 et la deuxième portion 22 peuvent avoir, dans des plans parallèles au plan du substrat, des sections identiques ou au contraire différentes, par exemple de forme circulaire, carrée, rectangulaire, hexagonale, etc.

De préférence, l'électrode 20 est symétrique par rapport à un plan parallèle au plan du substrat. La première portion 21 et la deuxième portion 22 ont alors une forme de section et des dimensions identiques. Ainsi, la deuxième portion 22 a pour effet de rendre les contacts électriques (d'entrée et de sortie) de l'électrode traversante 20 symétriques. L'avantage est que la structure d'interconnexion 1 peut ainsi transmettre une image électrique appliquée sur la première face 11 du substrat vers la deuxième face 12 du substrat 10 avec une fidélité spatiale et électrique améliorée.

De préférence, la portion intérieure 23 de l'électrode traversante 20 est une portion cylindrique creuse 231 et la structure d'interconnexion 1 comprend en outre un noyau 232 transparent disposé à l'intérieur de la portion cylindrique creuse 231 de chaque électrode traversante 20.

La portion cylindrique creuse 231 comprend une paroi constituée d'une couche mince d'un matériau optiquement transparent et électriquement conducteur, de préférence choisi parmi les matériaux optiquement transparents et électriquement conducteurs cités précédemment Encore plus avantageusement, le matériau optiquement transparent et électriquement conducteur formant la portion cylindrique creuse 231 est un oxyde métallique transparent tel que le SnO2. Ce matériau est avantageusement le même que celui utilisé pour réaliser la première portion 21 et/ou la deuxième portion 22. L'épaisseur e4 de la paroi de la portion cylindrique creuse 232, mesurée dans un plan parallèle au plan du substrat, peut être comprise entre 5 nm et 100 nm. La paroi est reliée à la première portion 21 et à la deuxième portion 22 de sorte que l'électrode est conductrice. La portion cylindrique 231 peut avoir une section circulaire, hexagonale, rectangulaire, etc.

Dans le premier mode de réalisation (cf. Fig.2), le noyau transparent 232 est formé d'un matériau électriquement conducteur et optiquement transparent. De préférence, le noyau transparent 232 est formé d'un matériau polymère transparent, par exemple le PEDOT. En effet, le PEDOT fait partie des matériaux optiquement transparents présentant les meilleures conductivités ionique et électronique. La conductivité de l'électrode traversante 20 est ainsi renforcée tout en préservant sa transparence. Ce mode de réalisation est particulièrement avantageux lorsque les tensions en jeu sont de l'ordre du µV. Dans ce premier mode de réalisation, on considère que le noyau transparent 232 fait partie de l'électrode traversante 20 qui, alors, est constituée au maximum de quatre éléments, ces quatre éléments étant la première portion 21, la deuxième portion 22, la portion cylindrique creuse 231 et le noyau transparent 232.

Dans le deuxième mode de réalisation (cf. Fig.3), le noyau transparent 232 est formé d'un deuxième matériau électriquement isolant et optiquement transparent. De préférence, le noyau transparent 232 est formé d'une colle de grade optique, par exemple une colle Vitralit^{™} 6127, 6128 ou équivalent, ou d'une résine de type époxy. Ces types de matériaux ont un coefficient de transmission supérieur à celui des matériaux polymères conducteurs tels que le PEDOT. La transparence de l'électrode traversante 20 est ainsi renforcée tout en préservant une conductivité acceptable. Ce mode de réalisation est particulièrement avantageux lorsque les ondes lumineuses transmises par la structure d'interconnexion 1 sont de faible luminance (inférieures à 50 cd/m²). Dans ce deuxième mode de réalisation, on considère que le noyau transparent 232 ne fait pas partie de l'électrode traversante 20 qui, alors, est constituée au maximum de trois éléments, ces trois éléments étant la première portion 21, la deuxième portion 22, et la portion cylindrique creuse 231.

Selon des variantes de réalisation non représentées, l'électrode 20 ne comprend que la portion intérieure 23, ou que la portion intérieure 23 et la première portion 21, ou que la portion intérieure 23 et la deuxième portion 22.

En référence à la figure 1, les électrodes transparentes 20 sont avantageusement réparties selon les deux directions du plan du substrat.

Sur la première face 11 et la deuxième face 12 du substrat 10, les électrodes transparentes 20 sont espacées deux à deux d'une distance D1, mesurée bord à bord, comprise entre 1 µm et 28 µm selon la direction X et d'une distance D2, mesurée bord à bord, comprise entre 1 µm et 28 µm selon la direction Y. Les distances D1 et D2 peuvent être identiques ou différentes. Les distances D1 et D2 sont inférieures à la distance D3 précédemment décrite du fait que les premières et deuxièmes portions 21-22 ont des dimensions latérales supérieures aux dimensions latérales de la portion intérieure 23.

Avantageusement les électrodes traversantes 20 sont toutes de forme et de dimensions identiques et présentent un premier pas de répétition P1 dans la première direction (ici suivant l'axe X) et un deuxième pas de répétition P2 dans une deuxième direction sécante à la première direction (ici suivant l'axe Y perpendiculaire à l'axe X).

De préférence, les pas de répétition P1 et P2 sont micrométriques, plus précisément P1 et P2 sont compris entre 7 µm et 83 µm. Les électrodes traversantes 20 sont ainsi préférentiellement espacées régulièrement et disposées en lignes et en colonne de sorte à former une matrice électrique de haute densité (> 400 électrodes par mm²) et avec une résolution spatiale micrométrique. Les pas de répétition P1 et P2 sont encore plus avantageusement identiques, évitant une distorsion spatiale des tensions transmises.

Les électrodes traversantes 20 peuvent ne pas couvrir toute la surface du substrat 10, et être arrangées en groupes séparés par des portions de substrat 10, ou encore être disposées de façon plus ou moins dense selon les régions du substrat 10 (non représenté).

Un deuxième aspect de l'invention concerne un procédé de fabrication 400 de la structure d'interconnexion 1.

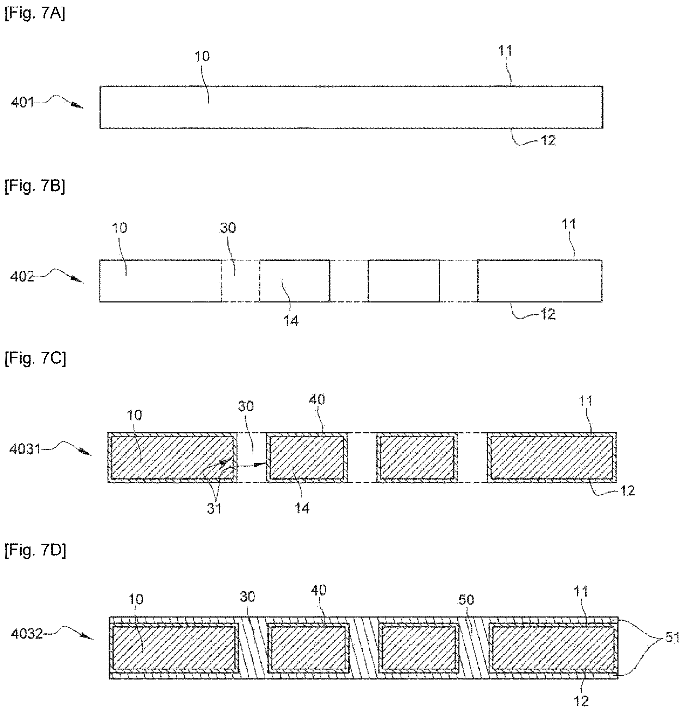

La figure 4 est un schéma synoptique illustrant l'enchaînement des étapes 401 à 403 du procédé de fabrication 400 de la structure d'interconnexion 1. Ces mêmes étapes 401 à 403 sont illustrées par les figures 7A à 7C, grâce à des vues de coupe schématique de la structure d'interconnexion 1.

En référence à la figure 7A, la première étape 401 consiste à fournir le substrat 10 formé du premier matériau électriquement isolant et optiquement transparent.

La deuxième étape 402 est illustrée en figure 7B et consiste à aménager dans le substrat une pluralité de cavités 30, dites cavités traversantes 30, traversant le substrat 10 entre la première face 11 et la deuxième face 12 du substrat 10, les cavités traversantes 30 étant espacées les unes des autres par une portion du substrat 10.

Les cavités sont par exemple formées par action mécanique (perçage) par jet d'eau haute pression, ou par photolithographie suivi d'une gravure (chimique ou sèche). Les cavités peuvent aussi être formées par moulage, notamment lorsque le substrat 10 est un substrat en polydiméthylsiloxane (ou PDMS).

La troisième étape 403 consiste à former dans les cavités traversantes 30 les électrodes transparentes 20, lesdites électrodes transparentes 20 traversant le substrat 10 de la première face 11 jusqu'à la deuxième face 12 du substrat 10, et étant isolées électriquement les unes des autres par le premier matériau électriquement isolant et optiquement transparent, précisément par la portion 14 du substrat 10.

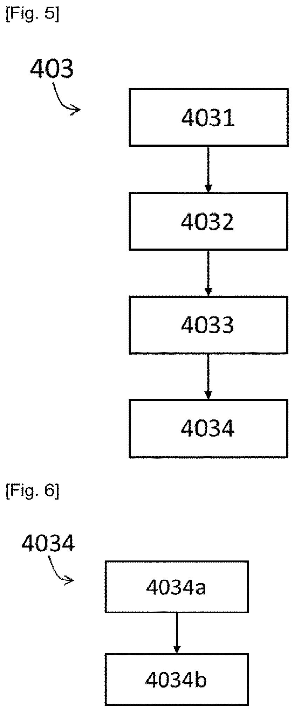

De préférence, la troisième étape 403 comprend les sous-étapes 4031 à 4033. L'enchainement de ces sous-étapes est représenté figure 5.

Les figures 7C à 7E représentent en vue de coupe schématique ces sous-étapes 4031 à 4033.

En référence à la figure 7C, la première sous-étape 4031 de l'étape 403 consiste à déposer une première couche 40 de matériau électriquement conducteur et optiquement transparent sur :
- la surface interne 31 de chacune des cavités traversantes 30,
- la première face 11 du substrat 10, et
- la deuxième face 12 du substrat 10.

De préférence, le dépôt est réalisé grâce à une technique de dépôt conforme tel que le dépôt par couches atomiques (ou ALD, pour « Atomic Layer Déposition » en anglais). Le SnO₂, le ZnO ou l'AZO sont des exemples de matériaux compatibles avec ce type de dépôt. De préférence, le matériau électriquement conducteur et optiquement transparent est donc du SnO₂, du ZnO ou de l'AZO

En référence à la figure 7D, la deuxième sous-étape 4032 de l'étape 403 consiste à remplir les cavités traversantes 30 avec un matériau transparent 50 et à former une surépaisseur 51 du matériau transparent 50 sur la première 11 et la deuxième face 12 du substrat.

Le matériau transparent peut être conducteur ou isolant. Des exemples de matériaux ont été donnés précédemment en relation avec les figures 1 et 2.

Le remplissage des cavités 30 par le matériau transparent 50 peut être réalisé par capillarité ou par aspiration. Par exemple, le matériau 50 est dispensé sur le substrat 10 et une différence de pression statique, due à la capillarité du matériau 50 dans les cavités 30 ou à un pompage (au moyen d'une pompe), permet de remplir les cavités 30. Une autre méthode de remplissage consiste à déposer dans une étuve le substrat 10 sur un bain de matériau transparent 50, et à mettre l'étuve sous vide puis à pression atmosphérique. Le remplissage des cavités 30 par le matériau transparent 50 peut aussi être réalisé par sérigraphie ou par dépôt localisé par jet d'encre ou spray de gouttelettes du matériau transparent 50 sur les cavités 30.

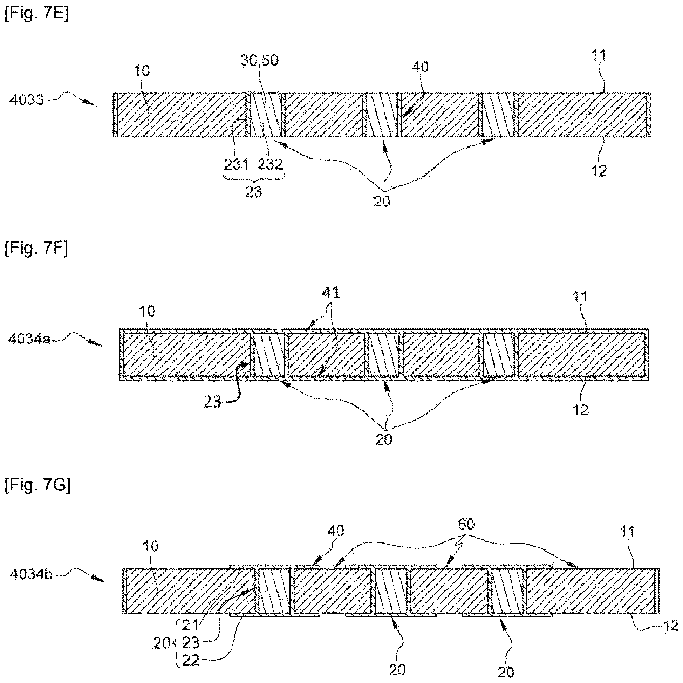

En référence à la figure 7E, la troisième sous-étape 4033 de l'étape 403 consiste à retirer la surépaisseur 51 du matériau transparent 50 ainsi que la première couche 40 de matériau électriquement conducteur et optiquement transparent déposée sur la première et deuxième face 11-12 du substrat 10.

Ce retrait peut être accompli par gravure, par exemple par gravure ionique réactive (RIE, acronyme de « Reactive Ion Etching » en anglais) ou par gravure par faisceaux d'ions (IBE, acronyme de « Ion Beam Etching » en anglais), ou par polissage mécano-chimique (ou CMP, pour « Chemical Mechanical Polishing » en anglais).

A l'issue de la sous-étape 4033, une pluralité d'électrodes transparentes et traversantes 20 ont été réalisées dans le substrat 10 transparent et isolant. Chaque électrode 20 comprend, à l'intérieur du substrat, la portion cylindrique creuse 231 (formée par la portion restante de la première couche 40) et le noyau 232 transparent disposé à l'intérieur de la portion cylindrique creuse 231 (formé par le matériau 50).

En référence à la figure 4, l'étape 403 de formation des électrodes transparentes et traversantes 20 peut aussi comprendre une sous-étape supplémentaire 4034, ou quatrième sous-étape 4034, de formation, pour chacune des électrodes traversantes 20 formées, d'une première portion d'électrode transparente 21 sur la première face 11 du substrat 10 et d'une deuxième portion d'électrode transparente 22 sur la deuxième face 12 du substrat 10.

La sous-étape 4034 comprend les opérations 4034a et 4034b dont l'enchainement est représenté figure 6.

La première opération 4034a, illustrée par la figure 7F, consiste à déposer une deuxième couche 41 de matériau électriquement conducteur et optiquement transparent sur la première face 11 et la deuxième face 12 du substrat 10. Le matériau électriquement conducteur et optiquement transparent formant la deuxième couche 41 est avantageusement du SnO₂, mais peut-être tout type de matériau conducteur et optiquement transparent (ITO, SnO2, ZnO, AZO, etc.).

La deuxième sous sous-étape 4034b est représentée figure 7G.

La deuxième opération 4034b de la sous-étape 4034, représentée par la figure 7G, consiste à graver la deuxième couche 41 de matériau électriquement conducteur et optiquement transparent dans des régions 60 des première et deuxième faces 11-12 du substrat, les régions 60 étant situées autour des électrodes transparentes 20.

A l'issue de la sous-étape 4034, les électrodes traversantes 20 comprennent, en plus de la portion intérieure 23 réalisée aux sous-étapes 4031 et 4032 précédentes, la première portion 21 et la deuxième portion 22.

Ces deux portions permettent de combler les irrégularités de surface après la sous-étape de dépôt 1032 et/ou la sous-étape de retrait 4033, tels que creux ou vides se formant sur les première et deuxième faces 11,12 du substrat 10. Ces irrégularités sont dues à un effet de capillarité, et s'observent en particulier lorsque les cavités ont un fort ratio d'aspect. C'est notamment le cas lorsque le substrat a une épaisseur E de 300 µm et les cavités ont des dimensions latérales micrométriques.

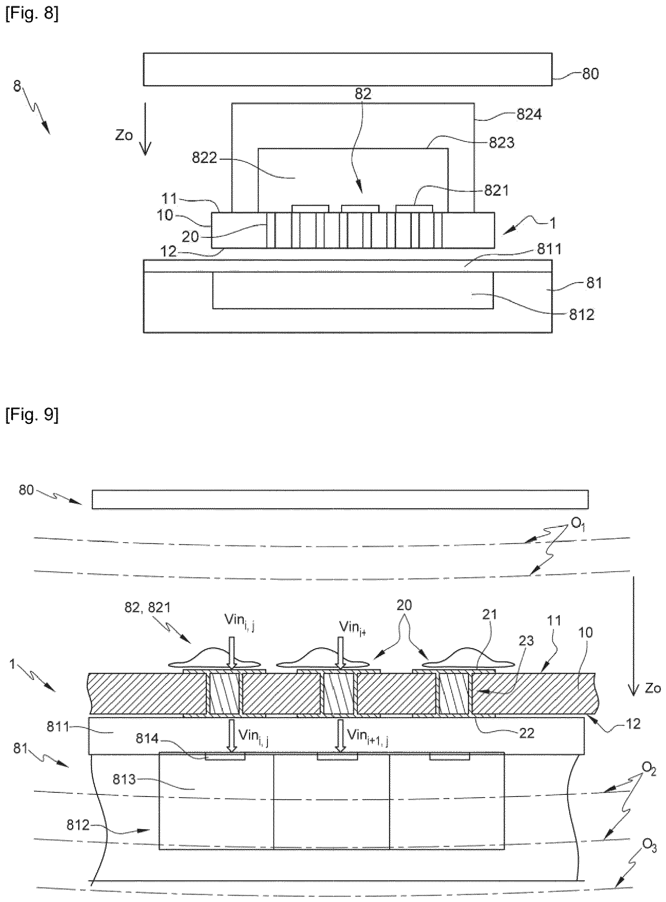

Un troisième aspect de l'invention concerne un système d'analyse d'un échantillon, ce système comprenant la structure d'interconnexion 1 décrite précédemment. La figure 8 représente schématiquement un mode de réalisation de ce système d'analyse 8, prêt à analyser un échantillon 82.

L'échantillon 82 peut être un échantillon biologique que l'on souhaite caractériser. Il peut s'agir de particules biologiques dans un milieu de culture ou dans un liquide corporel. Par particule biologique, on entend par une cellule, une bactérie ou autre micro-organisme de taille comprise entre 1 µm et 500 µm.

L'échantillon 82 est par exemple des cellules neuronales 821, ou neurones, dans un milieu de culture 822. De telles cellules ont une taille généralement comprise entre 15 µm et 120 µm. Elles se comportent comme des sources électriques aptes à produire un signal électrique, dit potentiel évoqué ou signal électrique Vin de l'échantillon 82. Le signal électrique Vin de l'échantillon 82 est de l'ordre du mV ou du µV.

Le système 8 est alors destiné à réaliser une imagerie de l'activité électrique des neurones avec une résolution spatiale à l'échelle d'un neurone et un champ de vue de l'ordre du cm². Le système 8 peut ainsi être destiné à la compréhension de certaines maladies neurodégénératives telles que Parkinson ou Alzheimer en permettant de visualiser en temps réel l'activité électrique neuronale et en permettant de suivre en parallèle l'effet de plusieurs traitements sur cette activité électrique.

Le système 8 comprend une source de lumière 80, la structure d'interconnexion 1, et un dispositif électro-optique 81.

La figure 9 représente schématiquement une vue agrandie de la structure d'interconnexion 1 dans le système 8 et l'échantillon 82 disposé sur la structure d'interconnexion 1.

La source de lumière 80 est apte à produire une onde lumineuse O1, dite onde lumineuse incidente O1, en direction de l'échantillon 82, selon une direction de propagation Zo.

La source de lumière 80 émet dans la bande spectrale s'étendant entre 400 nm et 800 nm, dite bande visible. Dans le cas où la source de lumière comporte plusieurs sources élémentaires de type diodes électroluminescentes (LED, acronyme de « Light Emitting Diodes » en anglais) ou de type diodes laser, les sources élémentaires émettent préférentiellement dans la même bande visible.

La source de lumière 82 peut comprendre un diaphragme, un filtre, ou un diffuseur (non représentés).

La source de lumière 82 peut comprendre un polariseur (non représenté).

La structure d'interconnexion 1 est disposée en regard de la source de lumière 80.

L'échantillon 82 est disposé sur la première face 11 du substrat 10 de la structure d'interconnexion 1.

En référence à la figure 8, l'échantillon 82 est plus particulièrement disposé en une couche 2D de neurones 821 sur la première face 11 du substrat 10. Les neurones 821 sont par ailleurs disposés dans le volume 823 d'une chambre fluidique 824, le volume 823 de la chambre fluidique 824 étant rempli d'un liquide, ou milieu de culture 822, circulant ou non et permettant de cultiver les neurones 821. La chambre fluidique 824 est réalisée dans un matériau optiquement transparent, par exemple en polydiméthylsiloxane ou PDMS. Elle a de préférence des dimensions micrométriques de sorte que la structure d'interconnexion 1 peut recevoir plusieurs chambres fluidiques.

La structure d'interconnexion 1 est suffisamment rigide pour recevoir l'échantillon 82.

Le dispositif électro-optique 81 est disposé en regard de la deuxième face 12 du substrat 10 de la structure d'interconnexion 1.

Par dispositif électro-optique, on désigne un dispositif optique comprenant au moins un élément à commande de signal présentant un effet électro-optique, cet élément à commande de signal étant utilisé pour moduler un faisceau de lumière traversant le dispositif 81.

En référence à la figure 9, le dispositif électro-optique 81 peut comprendre un film d'interface 811, par exemple une couche de polyamide, disposée entre la structure d'interconnexion 1 et l'élément à commande de signal 813 présentant un effet électro-optique.

L'élément à commande de signal 813 présentant un effet électro-optique est de préférence un cristal liquide. Un cristal liquide a la propriété de moduler la polarisation de la lumière qui le traverse sous l'effet d'un signal électrique qui lui est appliqué via une électrode de commande.

Dans l'exemple de réalisation donné figures 8 et 9, le dispositif électro-optique 81 est une cellule de cristaux liquides 812 dont les électrodes de commande 814 sont disposées en regard des électrodes traversantes 20 de la structure d'interconnexion 1. Chaque cristal liquide 813 de la cellule de cristaux liquides 812 est commandé par le signal électrique Vin émis par une cellule 821 de l'échantillon 82 et transmis par la structure d'interconnexion 1. Sous l'effet de ce signal électrique Vin, le cristal liquide 813 module localement l'onde lumineuse transmise O2 par l'échantillon et par la structure d'interconnexion 1. L'onde lumineuse modulée localement Os contient l'information de l'activité électrique de l'échantillon 82.

Un analyseur de lumière comprenant un capteur d'image et un polariseur (non représentés) peut être utilisé pour enregistrer l'image de l'onde lumineuse transmise et modulée Os par le système 8.

Le signal électrique Vin de l'échantillon est transmis directement et fidèlement, c'est-à-dire en respectant la distribution spatiale dudit signal électrique, à travers la structure d'interconnexion 1, cela grâce aux électrodes traversantes 20 de la structure d'interconnexion 1.

## Revendications

1. Structure d'interconnexion (1) comprenant :
- un substrat (10) formé d'un premier matériau électriquement isolant et optiquement transparent, le substrat (10) comprenant une première face (11) et une deuxième face (12) opposée, la première face (11) définissant un plan du substrat ;
- une pluralité d'électrodes transparentes (20) ;
ladite structure d'interconnexion (1) étant **caractérisée en ce que** les électrodes transparentes (20) traversent le substrat (10) de la première face (11) jusqu'à la deuxième face (12) du substrat (10) parallèlement les unes aux autres, et sont isolées électriquement les unes des autres par le premier matériau électriquement isolant et optiquement transparent.

2. Structure d'interconnexion (1) selon la revendication 1, dans laquelle chaque électrode transparente (20) comprend une première portion (21) disposée sur la première face (11) du substrat (10).

3. Structure d'interconnexion (1) selon la revendication 2, dans laquelle chaque électrode transparente (20) comprend en outre une deuxième portion (22) disposée sur la deuxième face (12) du substrat (10) en regard de la première portion (21).

4. Structure d'interconnexion (1) selon la revendication 3, dans laquelle les électrodes transparentes (20) sont symétriques par rapport à un plan parallèle au plan du substrat.

5. Structure d'interconnexion (1) selon l'une quelconque des revendications 1 à 4, dans laquelle chaque électrode transparente (20) comprend une portion cylindrique creuse (231) s'étendant de la première face (11) jusqu'à la deuxième face (12) du substrat (10), la structure d'interconnexion (1) comprenant en outre un noyau transparent (232) disposé à l'intérieur de la portion cylindrique creuse (231) de chaque électrode transparente (20).

6. Structure d'interconnexion (1) selon la revendication 5, dans laquelle le noyau transparent (232) est formé d'un matériau électriquement conducteur et optiquement transparent.

7. Structure d'interconnexion (1) selon la revendication 5, dans laquelle le noyau transparent (232) est formé d'un deuxième matériau électriquement isolant et optiquement transparent.

8. Structure d'interconnexion (1) selon l'une quelconque des revendications 5 à 7, dans laquelle la portion cylindrique creuse (231) présente dans un plan parallèle au plan du substrat des dimensions extérieures comprises entre 5 µm et 50 µm, et comprend une paroi d'épaisseur (e4) comprise entre 5 nm et 100 nm.

9. Structure d'interconnexion (1) selon l'une quelconque des revendications 1 à 8, dans laquelle le substrat (10) présente entre la première face (11) et la deuxième face (12) du substrat (10) une épaisseur (E) comprise entre 50 µm et 300 µm.

10. Structure d'interconnexion (1) selon l'une quelconque des revendications 1 à 9, dans laquelle les électrodes transparentes (20) sont espacées les unes des autres à l'intérieur du substrat (10) par une distance bord-à-bord (D3) comprise entre 5 µm et 30 µm.

11. Structure d'interconnexion (1) selon l'une quelconque des revendications 1 à 10, dans laquelle les électrodes transparentes (20) sont identiques en taille et en forme et présentent dans le plan du substrat un premier pas de répétition (P1) dans une première direction (X) et un deuxième pas de répétition (P2) dans une deuxième direction (Y) sécante à la première direction (X).

12. Structure d'interconnexion (1) selon l'une quelconque des revendications 1 à 11, dans laquelle les électrodes transparentes (20) sont formées d'un polymère conducteur transparent ou d'un oxyde métallique conducteur transparent tel que le dioxyde d'étain.

13. Procédé (400) de fabrication d'une structure d'interconnexion (1) comprenant les étapes suivantes :
- Fournir (401) un substrat (10) formé d'un premier matériau électriquement isolant et optiquement transparent, le substrat (10) comprenant une première face (11) et une deuxième face (12) opposée, la première face (11) définissant un plan du substrat ;
- Aménager (402) dans le substrat (10) une pluralité de cavités (30), dites cavités traversantes, traversant le substrat (10) entre la première face (11) et la deuxième face (12) du substrat (10) parallèlement les unes aux autres, et étant espacées les unes des autres ;
- Former (403) dans les cavités traversantes (30) une pluralité d'électrodes transparentes (20), lesdites électrodes transparentes (20) traversant le substrat (10) de la première face (11) jusqu'à la deuxième face (12) du substrat (10) parallèlement les unes aux autres, et étant isolées électriquement les unes des autres par le premier matériau électriquement isolant et optiquement transparent.

14. Procédé (400) de fabrication d'une structure d'interconnexion (1) selon la revendication 13, dans lequel l'étape de formation (403) de la pluralité d'électrodes transparentes (20) comprend les étapes suivantes :
- Déposer (4031) une première couche (40) de matériau électriquement conducteur et optiquement transparent sur :
∘ la surface interne (31) de chacune des cavités traversantes (30),
∘ la première face (11) du substrat (10), et
∘ la deuxième face (12) du substrat (10) ;
- Remplir (4032) les cavités traversantes (30) avec un matériau transparent (50) et former une surépaisseur (51) du matériau transparent (50) sur la première (11) et la deuxième face (12) du substrat (10) ;
- Retirer (4033) la surépaisseur (51) du matériau transparent (50) ainsi que des portions de la première couche (40) de matériau électriquement conducteur et optiquement transparent déposées sur la première face (11) et la deuxième face (12) du substrat (10).

15. Procédé (400) de fabrication d'une structure d'interconnexion (1) selon la revendication 14, dans lequel l'étape de formation (403) des électrodes transparentes (20) comprend une étape supplémentaire (4034) de formation, pour chacune des électrodes traversantes (20) formées, d'une première portion (21) d'électrode transparente sur la première face (11) du substrat (12) et d'une deuxième portion (22) d'électrode transparente sur la deuxième face (12) du substrat (10), ladite étape supplémentaire (4034) comprenant les sous-étapes suivantes :
- déposer (4034a) une deuxième couche (41) de matériau électriquement conducteur et optiquement transparent sur la première face (11) et la deuxième face (12) du substrat (10),
- graver (4034b) la deuxième couche (41) de matériau électriquement conducteur et optiquement transparent dans des régions (60) de la première (11) et deuxième face (12) du substrat (10), les régions (60) étant situées autour des électrodes transparentes (20) formées.

16. Système (8) d'analyse d'un échantillon comprenant :
- une source de lumière (80) produisant une onde lumineuse (O1), dite onde lumineuse incidente (O1),
- une structure d'interconnexion (1) selon l'une quelconque des revendications 1 à 12, la première face (11) du substrat (10) étant disposée en regard de la source de lumière (80) et étant destinée à recevoir un échantillon (82) comprenant au moins une source (821) apte à produire un signal électrique (Vin), dit signal électrique de l'échantillon (Vin), le signal électrique (Vin) de l'échantillon (82) étant transmis de la première face (11) du substrat (10) jusqu'à la deuxième face (12) du substrat (10) par l'électrode traversante (20) située en regard de ladite source (821),
- un dispositif électro-optique (81) disposé en regard de la deuxième face (12) du substrat (10),
le dispositif électro-optique (81) présentant une pluralité d'électrodes de commande (814) disposées en regard de la pluralité d'électrodes traversantes (20) de la structure d'interconnexion (1), chaque électrode de commande (814) de la pluralité d'électrodes de commande (814) étant connectée à une électrode traversante (20) de la pluralité d'électrodes traversantes (20), de sorte que le dispositif électro-optique (81) module l'onde lumineuse transmise (O2) par l'échantillon (82) sous l'effet du signal électrique de l'échantillon (Vin).

17. Système d'analyse selon la revendication 16, dans lequel le dispositif électro-optique (81) est une cellule de cristaux liquides (812).
